# EUROPÄISCHE PATENTSCHRIFT

(11) **EP 1 807 112 B1**
(45) Veröffentlichungstag und Bekanntmachung des Hinweises auf die Patenterteilung: **09.12.2009**
(21) Anmeldenummer: 05801421.8
(22) Anmeldetag: 28.10.2005
(51) Int. Cl.: A61K 47/12, A61K 31/282

(54) **STABILE WÄSSRIGE FORMULIERUNGEN EINES PLATIN DERIVATS**
STABLE AQUEOUS FORMULATION OF A PLATIN DERIVATIVE
PREPARATIONS AQUEUSES STABLES D'UN DERIVE DE PLATINE

(30) Priorität: 02.11.2004 DE 102004052877
(43) Veröffentlichungstag der Anmeldung: 18.07.2007
(73) Patentinhaber: Ebewe Pharma Ges.m.b.H. Nfg. KG, 4866 Unterach (AT)
(72) Erfinder: RAMETSTEINER, Reinhard, A-4866 Unterach (AT); SCHNAIT, Heinz, A-4863 Seewalchen (AT)
(74) Vertreter: Landgraf, Elvira
(86) Internationale Anmeldenummer: PCT/EP2005/011570
(87) Internationale Veröffentlichungsnummer: WO 2006/048194

(56) Entgegenhaltungen:
- EP-A- 0 774 963
- EP-A- 0 943 331
- WO-A-01/15691
- WO-A-03/047587
- DE-A1- 10 314 377
- US-A1- 2003 109 515

## Beschreibung

Die Erfindung betrifft stabile wässrige Formulierungen eines Platin Derivats, insbesondere von Oxaliplatin, sowie deren pharmazeutische Anwendung als Arzneimittel.

Oxaliplatin ist ein antineoplastischer Wirkstoff, der aus der US 4 169 846 bekannt ist. Oxaliplatin wird alleine oder in Kombination mit 5-FU und Folinat vor allem zur Behandlung von metastasiertem Dickdarm-Mastdarmkrebs eingesetzt und bevorzugt parenteral appliziert.

Aus der EP 774 963 A sind pharmazeutisch stabile Zubereitungen von Oxaliplatin für die parenterale Anwendung bekannt. Die beschriebene Lösung von Oxaliplatin in Wasser weist einen pH-Wert von 4.5 bis 6 auf und soll frei von jedem sauren oder alkalischen Zusatz oder Puffermittel oder anderem Zusatzstoff sein.

Aus der WO 03/047587 A ist eine Formulierung von Oxaliplatin mit einer effektiven stabilisierenden Menge Milchsäure bzw. deren Salzen bekannt. Dabei wird in den Beispielen eine Konzentration von 0.001 M bis 0.0005 M an Stabilisierungsmittel zur Herstellung der Formulierung verwendet.

Aus der EP 943 331 A sind Formulierungen von Oxaliplatin bekannt, die einen Pufferzusatz ausgewählt aus Oxalsäure oder einem Alkalimetallsalz der Oxalsäure enthalten.
Dabei wird die stabilisierende Wirkung von Oxalsäure bzw. Alkalimetallsalzen der Oxalsäure der Verwendung anderer üblicher Puffersubstanzen gegenübergestellt..
Als übliche Puffersubstanzen werden dabei Citrat, Natriumacetat, Tris, Glycin und Phosphat jeweils in einer Konzentration von 0.1 M verwendet.

Aufgabe der Erfindung war es eine stabile wässrige Formulierung von Oxaliplatin bereitzustellen, die gegenüber den bekannten wässrigen Formulierungen eine verbesserte Stabilität aufweisen.

Die Aufgabe konnte überraschenderweise gelöst werden, dass eine stabile wässrige Formulierung von OXaliplatin durch Zugabe von Natriumacetat in einer sehr geringen Konzentration erhalten wird.

Gegenstand der Erfindung ist daher eine stabile wässrige Lösung von Oxaliplatin, dadurch gekennzeichnet, dass die wässrige Lösung als stabilisierendes Additiv Natriumacetat in einer Konzentration von 0.005 bis 0.00005 M aufweist.

Natriumacetat weisen im Konzentrationsbereich von 0.005 bis 0.00005 M ausgezeichnete stabilisierende Wirkung der wässrigen Oxaliplatinlösung auf. Bevorzugt enthält die wässrige Oxaliplatinlösung eines der stabilisierenden Additive in einer Konzentration von 0.0001 bis 0.001 M.

Der Gehalt an Oxaliplatin in der erfindungsgemäßen wässrigen stabilen Formulierung kann 0.1 - 10 mg/ml, vorzugsweise 2- 5 mg/ml betragen.

Bevorzugt weist die stabile wässrige Formulierung einen pH-Wert von 4 bis 6 auf.

Die erfindungsgemäßen stabilen wässrigen Formulierungen weisen nach einer Lagerzeit von 3 Monaten, bei 45°C und 75 % rel. Feuchtigkeit mindestens 95% des ursprünglichen Gehalts an Oxaliplatin auf.

Die Herstellung der erfindungsgemäßen Zubereitung erfolgt durch Auflösen von Oxaliplatin in Wasser oder in einer wässrigen Lösung des stabilisierenden Additivs unter Rühren. Erfolgt die Auflösung in Wasser ist die Zugabe des stabilisierenden Additivs als zusätzlicher Herstellungsschritt notwendig. Anschließend erfolgt die Einstellung des pH-Wertes. Die Lösung wird abschließend mittels geeigneter Sterilisationsmethode sterilisiert und in entsprechende Behältnisse abgefüllt (gegebenenfalls unter Schutzgasatmosphäre). Die eingesetzten Behältnisse wurden vorher gereinigt und sterilisiert.

Die erfindungsgemäßen stabilen wässrigen Formulierungen von Oxaliplatin sind zur parenteralen Verabreichung als "ready to use" Formulierungen vorgesehen und können nach Verdünnung mit gängigen Infusionsmedien, wie z.B. 5 % Glukose mit Hilfe herkömmlicher Instrumente verabreicht werden.

### Beispiele:

### Vergleichsbeispiel 1: Lösung mit Phosphat

40 mg Oxaliplatin werden in einem 20 ml Messkolben mit 1 ml Phosphatlösung (89.1 mg Na₂HPO₄.2H₂O in 50 ml gereinigtem Wasser gelöst; entspricht 0.01 mol/l) versetzt und mit gereinigtem Wasser auf 20 ml aufgefüllt. Anschließend wird der pH-Wert mittels 10% Phosphorsäure auf 5.0 eingestellt. Diese Lösung wird anschließend über einen 0.2 µm Spritzenfilter filtriert, zu je 2 ml in 5 ml Vials abgefüllt, verbördelt und unter den unten angegebenen Bedingungen gelagert.

### Vergleichsbeispiel 2: Lösung mit Citrat

40 mg Oxaliplatin werden in einem 20 ml Messkolben mit 1 ml Citratlösung (148.0 mg Natriumcitrat.2H₂O in 50 ml gereinigtem Wasser gelöst; entspricht 0.01 mol/l) versetzt und mit gereinigtem Wasser auf 20 ml aufgefüllt. Anschließend wird der pH-Wert mittels 10 % Citronensäure auf 5.0 eingestellt. Diese Lösung wird anschließend über einen 0.2 µm Spritzenfilter filtriert, zu je 2 ml in 5 ml Vials abgefüllt, verbördelt und unter den unten angegebenen Bedingungen gelagert.

### Vergleichsbeispiel 3: Lösung mit Phosphat

40 mg Oxaliplatin werden in einem 20 ml Messkolben mit 1 ml Phosphatlösung (89.1 mg Na₂HPO₄.2H₂O in 50 ml gereinigtem Wasser gelöst; entspricht 0.01 mol/l) versetzt und mit gereinigtem Wasser auf 20 ml aufgefüllt. Anschließend wird der pH-Wert mittels 10 % Phosphorsäure auf 4.0 eingestellt. Diese Lösung wird anschließend über einen 0.2 µm Spritzenfilter filtriert, zu je 2 ml in 5 ml Vials abgefüllt, verbördelt und unter den unten angegebenen Bedingungen gelagert.

### Beispiel 1: Lösung mit Acetat

80 ml gereinigtes Wasser werden in einem 100 ml Messkolben vorgelegt. Anschließend werden 0.0042 g Natriumacetat zugesetzt. Man gibt 500 mg Oxaliplatin zur Lösung und rührt die Lösung so lange bis Oxaliplatin vollständig gelöst ist. Anschließend wird der pH Wert mit 1 % Essigsäure auf 5.0 eingestellt. Nach Auffüllen des Messkolbens mit gereinigtem Wasser bis zur Marke wird die Lösung über einen 0.2 µm Spritzenfilter filtriert, zu je 2ml in 5 ml Vials abgefüllt, verbördelt und unter den unten angegebenen Bedingungen gelagert.

### Beispiel 2: Lösung mit Acetat

80 ml gereinigtes Wasser werden in einem 100 ml Messkolben vorgelegt. Anschließend werden 0.0042 g Natriumacetat zugesetzt. Man gibt 500 mg Oxaliplatin zur Lösung und rührt die Lösung so lange bis Oxaliplatin vollständig gelöst ist. Anschließend wird der pH Wert mit 1 % Essigsäure auf 6.0 eingestellt. Nach Auffüllen des Messkolbens mit gereinigtem Wasser bis zur Marke wird die Lösung über einen 0.2 µm Spritzenfilter filtriert, zu je 2ml in 5 ml Vials abgefüllt, verbördelt und unter den unten angegebenen Bedingungen gelagert.

Analog zu den hier ausgeführten Beispielen wurden Oxaliplatin Lösungen mit unterschiedlichen stabilisierendeN Additiven hergestellt und unter den in den folgenden Tabellen beschriebenen Bedingungen gelagert und getestet.

Die Untersuchung des Gehalts und der Reinheit erfolgte mittels HPLC an einer Octadecyl silica 5 µm Säule. Die Auswertung erfolgte mittels UV Detektion bei 210 nm und gegen Oxaliplatin als externer Standard. Ein Gehalt von 100 % entspricht einer Konzentration von 2 mg/ml Oxaliplatin.

Die Ergebnisse sind in den Tabellen 1-2 dargestellt

**Tab.1 Gehalt und Reinheit nach 4 Wochen Lagerung bei 40°C/75.r.F.**

| | **Gehalt** **Ausgang** | **Gehalt** **40°C / 4Wochen** |
|---|---|---|
| 0,5mM Citronensäure | 99.1% | 97.1% |
| 0,5mM Na-Acetat | 101.7% | 99.9% |
| 0,5mM Weinsäure | 99.7% | 97.2% |
| 0,5mM Phosphat | 97.6% | 96.8% |
| 0,5mM Bernsteinsäure | 99.5% | 96.1% |
| 0,5mM Maleinsäure | 101.8% | 98.7% |

**Tab. 2 Gehalt und Reinheit nach 3 Monaten Lagerung bei 40°C175.r.F**

| | **Gehalt** **Ausgang** | **Gehalt** **40°C / 3 Monate** |
|---|---|---|
| 0,5mM Na-Acetat | 101.7% | 99.5% |
| 0,5mM Weinsäure | 99.7% | 97.5% |
| 0,5mM Bernsteinsäure | 99.5% | 95.9% |
| 0,5mM Maleinsäure | 101.8% | 97.3% |

**Tab 3**

| **Gehalt nach 4 Wochen Lagerung bei 40°C/75.r.F** **Additiv = Natriumacetat; pH =5.5** | | | |
|---|---|---|---|
| **Tab. 3a** | | | |
| **Menge Additiv** | **Gehalt** **Ausgang** | **Gehalt** **4Wochen** | |
| 0.05 mM Na-Acetat | 100,90% | 99,40% | |
| 0.1 mM Na-Acetat | 99,60% | 97,90% | |
| 0.5 mM Na-Acetat | 101,70% | 99,90% | |
| 1 mM Na-Acetat | 101,20% | 99,10% | |
| 5 mM Na-Acetat | 100,30% | 98,20% | |
| ohne Additiv | 101,80% | 95,90% | |

| **Gehalt und Reinheit nach 3 und 6 Monaten bei 25°C/60%.r.F** **Additiv = Natriumacetat; pH = 5.5** | | | |
|---|---|---|---|
| **Tab. 3b** | | | |
| **Menge Additiv** | **Gehalt** **Ausgang** | **Gehalt** **3 Monate** | **Gehalt** **6 Monate** |
| 0.5 mM Na-Acetat | 100,10% | 99,60% | 99,50% |

Wie aus den Tabellen 1-3 hervorgeht, zeigt das erfindungsgemäß eingesetzte Natriumacetat bereits in einer geringen Konzentration von 0.0005 M eine ausgezeichnete stabilisierende Wirkung.

## Patentansprüche

1. Stabile wässrige Lösung von Oxaliplatin, **dadurch gekennzeichnet, dass** die wässrige Lösung als stabilisierendes Additiv Natriumacetat in einer Konzentration von 0.005 bis 0.00005 M aufweist.

2. Stabile wässrige Lösung nach Anspruch1, **dadurch gekennzeichnet, dass** das stabilisierende Additiv in einer Konzentration von 0.0001 bis 0.001 M vorliegt.

3. Stabile wässrige Lösung von Oxaliplatin nach einem der Ansprüche 1 bis 2, **dadurch gekennzeichnet, dass** der Gehalt an Oxaliplatin in der Lösung 0.1-10 mg/ml beträgt.

4. Stabile wässrige Lösung von Oxaliplatin nach Anspruch 3, **dadurch gekennzeichnet, dass** der Gehalt an Oxatiptatin in der Lösung 2-5 mg/ml beträgt.

5. Stabile wässrige Lösung von Oxaliplatin nach einem der Ansprüche 1 bis 4, **dadurch gekennzeichnet, dass** die Lösung einen pH-Wert von 4 bis 6 aufweist.

## Claims

1. Stable aqueous solution of oxaliplatin, **characterized in that** the aqueous solution exhibits, as stabilizing additive, sodium acetate in a concentration of 0.005 to 0.00005M.

2. Stable aqueous solution according to Claim 1, **characterized in that** the stabilizing additive is present in a concentration of 0.0001 to 0.001M.

3. Stable aqueous solution of oxaliplatin according to either of Claims 1 and 2, **characterized in that** the content of oxaliplatin in the solution is 0.1-10 mg/ml.

4. Stable aqueous solution of oxaliplatin according to Claim 3, **characterized in that** the content of oxaliplatin in the solution is 2-5 mg/ml.

5. Stable aqueous solution of oxaliplatin according to one of Claims 1 to 4, **characterized in that** the solution exhibits a pH value of 4 to 6.

## Revendications

1. Solution aqueuse stable d'oxaliplatine, **caractérisée en ce que** la solution aqueuse présente, en tant qu'additif de stabilisation, de l'acétate de sodium dans une concentration allant de 0,005 à 0,00005 M.

2. Solution aqueuse stable selon la revendication 1, **caractérisée en ce que** l'additif de stabilisation est présent dans une concentration allant de 0,0001 à 0,001 M.

3. Solution aqueuse stable d'oxaliplatine selon l'une quelconque des revendications 1 à 2, **caractérisée en ce que** la teneur en oxaliplatine dans la solution est de 0,1 -10 mg/ml.

4. Solution aqueuse stable d'oxaliplatine selon la revendication 3, **caractérisée en ce que** la teneur en oxaliplatine dans la solution est de 2 - 5 mg/ml.

5. Solution aqueuse stable selon l'une quelconque des revendications 1 à 4, **caractérisée en ce que** la solution présente une valeur pH allant de 4 à 6.
